# EUROPEAN PATENT APPLICATION

(11) **EP 0 852 949 A2**
(43) Date of publication of application: **15.07.1998**
(21) Application number: 98105789.6
(22) Date of filing: 30.03.1998
(51) Int. Cl.: A61K 31/195

(54) **Use of alpha-amino-acids for enhancing desmosomal degradation or stratum corneum desquamation**

(30) Priority: 31.03.1997 JP 94389/97; 27.08.1997 JP 244827/97
(71) Applicant: SHISEIDO COMPANY LIMITED, Chuo-ku, Tokyo 104-8010 (JP)
(72) Inventor: Masuda, Yoshiko, Yokohama-shi, Kanagawa-ken, 223-8553 (JP); Sato, Junko, Shiseido Research Center, Yokohama-shi, Kanagawa-ken, 236-8643 (JP); Koyama, Junichi, Shiseido Research Center, Yokohama-shi, Kanagawa-ken, 236-8643 (JP)
(74) Representative: Weisert, Annekäte, Dipl.-Ing. Dr.-Ing.

(57) **Abstract**

A cosmetic or dermatological topical preparation contains, as active ingredient, an α-amino acid derivative of the formula (I) 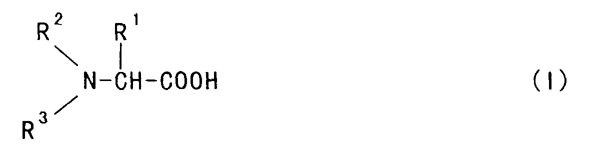 wherein R¹ to R³ represent specific organic radicals. This preparation is useful in enhancing the stratum corneum desquamation or desmosomal degradation of the skin and thereby restoring or maintaining a skin having a healthy and attractive appearance.

## Description

### BACKGROUND OF THE INVENTION

### 1. Field of the Invention

This invention relates to external preparations for application to the skin which contain an α-amino acid derivative as active ingredient, and more particularly to such preparations for enhancing the desmosomal degradation or stratum corneum desquamation of the skin. This invention is chiefly utilized in the fields of cosmetics and dermatology.

### 2. Description of the Prior Art

It has been demonstrated by previous studies that humectants act effectively on dry skin [see, for example, Tatsuya Ozawa et al, "The role of humectants in skin moisture retention", Hifu (SKIN RESEARCH), 27, 276-288 (1985)]. However, the mechanism on which a humectant improves desquamation as a characteristic symptom of dry skin has not been clearly understood. Nevertheless, a wide variety of humectants are actually used in cosmetic preparations for application to the skin and it has been confirmed that they produce a certain effect.

A widely used typical example of such humectants or emollients is lactic acid which comes into the category of α-hydroxycarboxylic acids. It is also known that, in addition to the aforesaid lactic acid coming into the category of α-hydroxycarboxylic acids, α-hydroxycarboxylic acids having a longer-chain alkyl group than methyl and α-hydroxycarboxylic acids also having a carboxyl substituent (e.g., citric acid and tartaric acid) may be incorporated especially in cosmetic preparations having the effect of softening the stratum corneum (Japanese Patent Laid-Open No. 8007/'83). Moreover, the use of 2-(or α-)hydroxycarboxylic acids or related compounds in compositions for alleviating dermatological symptoms of aging is also known (Japanese Patent Laid-Open No. 139947/'93). Furthermore, there has also been provided a composition for the treatment of dry skin which contains a specific hydroxycarboxylic acid or ketocarboxylc acid or an ester thereof and a lipid component such as ceramide (Japanese Patent Laid-Open No. 157283/'94).

Japanese Patent Laid-Open No. 8007/'83 suggests that α-hydroxycarboxylic acids have a softening effect on the skin because they improve the elastic modulus of a stratum corneum sheet. On the other hand, Japanese Patent Laid-Open No. 139947/'93 suggests that 2-(or α-)hydroxycarboxylic acids have the effect of decreasing the aggregation of corneocytes in the stratum corneum, but they are ineffective in the outer layer of the stratum corneum. Moreover, Japanese Patent Laid-Open No. 157283/'94 suggests that the above-described composition brings about a visual improvement of dry skin in in vivo potency tests.

Meanwhile, it has been generally believed that lipids take part in the adhesion of the stratum corneum (or a layered structure of keratinized cells). However, on the basis of information obtained by electron microscopy, a suggestion has recently been offered to the effect that the desmosome is an intrinsic structure for the adhesion of stratum corneum cells [see, for example, an article by Kitajima (Journal of Japanese Cosmetic Science Society, Vol. 15, No. 4 (1991), pp. 225-230]. Moreover, in the aforementioned article, Kitajima has also offered the presumption that the digestion of desmosomes by proteases is a key factor in stratum corneum desquamation. Furthermore, A. Lundstroem et al. have suggested that a chymotrypsin-like enzyme with a molecular weight of 25 kDa, which is considered to exist in the stratum corneum, plays a definite role in desquamation under in vivo conditions [Acta Derm Venereol (Stockh), 1991: 71: 471-474].

On the other hand, the present inventors made an investigation on stratum corneum desquamation and thereby demonstrated that, besides the aforesaid chymotrypsin-like enzyme, a trypsin-like enzyme with a molecular weight of about 30 kDa exists as an endogenous protease which may possibly participate in desquamation [Arch. Dermatol. Res. (1994), 286: 249-253]. Moreover, they also demonstrated that humectants can maintain a healthy skin by controlling the site (i.e., aqueous environment) required for the manifestation of the activities of the aforesaid two enzymes in the skin and, in particular, desmosomes [see, for example, FRAGRANCE JOURNAL (1995), 13-18].

In order that not only the skin of patients with dry skin, but also the skin of normal subjects may maintain a healthy and attractive appearance, it is necessary that the formation of the stratum corneum consisting of keratinized cells derived from dead epidermal cells be in harmony with its shedding by physiological desquamation.

It is certain that, as described above, humectants participate in stratum corneum desquamation and play a definite role in maintaining a healthy skin. However, in individuals showing a reduction in the aforesaid two types of enzyme activities, it might be impossible to achieve a sufficient degree of stratum corneum desquamation simply by controlling the environment for the manifestation of these activities with the aid of a humectant.

Accordingly, an object of the present invention is to provide a preparation which not only serves the purpose of moisture retention, but also can more positively enhance at least the aforesaid two types of enzyme activities themselves.

### SUMMARY OF THE INVENTION

The present inventors have now found that stratum corneum desquamation is brought about through the decomposition of proteins (at least desmoglein) in the stratum corneum by the aforesaid enzymes. This mechanism of stratum corneum desquamation forms a contrast to the mechanism of the compounds described in the aforementioned Japanese Patent Laid-Open No. 139947/'93 in which their effect does not depend on an action in the outer layer of the stratum corneum, and is hence entirely new information.

Moreover, the present inventors have also found that the degradation of desmosomes is enhanced by α-amino acids which need not necessarily have a free hydroxyl group, i.e., compounds having a carboxyl group and an amino group attached to the same carbon atom have the effect of enhancing the decomposition of desmoglein in the stratum corneum, thereby maintaining a skin having a healthy and attractive appearance, and/or preventing and decreasing the dullness of skin. Incidentally, the "dullness" may be defined as a state where the clearness of skin is dimmed.

Thus, according to the present invention, there is provided a preparation for enhancing the desmosomal degradation or stratum corneum desquamation of the skin which contains, as active ingredient, at least one α-amino acid derivative of the following formula (I), a salt thereof, or a carboxylic acid ester thereof.

Formula (I): 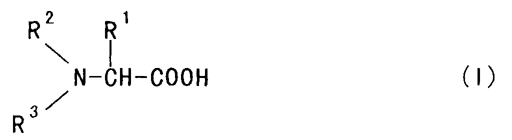
wherein R¹ is a hydrogen atom or an unsubstituted or substituted lower alkyl group, and the substituent in the substituted lower alkyl group is a hydroxyl group, a mercapto group which may optionally be substituted by a lower alkyl group, an amino group which may optionally be substituted by a lower alkyl group, a lower acyl group, an amidino group (-C(=NH)-NH₂), or an N-mono- or N,N'-di(lower alkyl)amidino group, a phenyl group which may optionally be substituted by a hydroxyl group, a five-membered heterocyclic group which has one or two nitrogen atoms in the ring and which may optionally have a benzene ring fused thereto, or a carbamoyl group (-CONH₂); and
R² and R³ are each independently a hydrogen atom, a lower alkyl group, or a lower acyl group; or
one of R² and R³ is combined with R¹ to form a propane-1,3-diyl, 2-hydroxypropane-1,3-diyl or 1-hydroxypropane-1,3-diyl group.

The compounds represented by the above formula (I), salts thereof or carboxylic acid esters thereof act so as to reduce the amount of residual desmoglein in stratum corneum sheets, for example, so as to accelerate the turnover of the stratum corneum and thereby provide a fresh and young skin condition. Accordingly, they can be used as active ingredients in external (or percutaneous) preparations for application to the skin, especially in cosmetic or dermatological preparations.

Moreover, the present invention also provides the use of a preparation containing at least one compound of formula (I) for the purpose of enhancing the stratum corneum desquamation or desmosomal degradation of the skin. Furthermore, the present invention also provides a method for restoring or maintaining a skin having a healthy and attractive appearance which comprises the step of topically applying an effective amount of at least one compound of formula (I) to the skin and thereby enhancing the stratum corneum desquamation or desmosomal degradation of the skin.

### DETAILED DESCRIPTION OF THE INVENTION

The term "lower alkyl" as used herein means strain-chain or branched alkyl groups of 1 to 6 carbon atoms. Specific examples of unsubstituted lower alkyl groups include, but are not limited to, methyl (related to alanine or its derivatives), ethyl, propyl, isopropyl (related to valine or its derivatives), 2-methylpropyl (related to leucine or its derivatives), 1-methylpropyl (related to isoleucine or its derivatives), n-butyl, n-pentyl and n-hexyl.

The substituted lower alkyl group represented by R¹ is a lower alkyl group as described above in which one or more hydrogen atoms are replaced by the same or different substituents that will be specifically described later.

Typical examples of substituted lower alkyl groups in which the substituent is a hydroxyl group include hydroxymethyl (related to serine or its derivatives) and 1-hydroxyethyl (related to threonine or its derivatives). Typical examples of substituted lower alkyl groups in which the substituent is a mercapto group that may optionally be substituted by a lower alkyl group include mercaptomethyl (related to cysteine or its derivatives) and 2-methylthioethyl (related to methionine or its derivatives).

Typical examples of substituted lower alkyl groups in which the substituent is an amino group that may optionally be substituted by a lower alkyl group (preferably methyl or ethyl), a lower acyl group (preferably formyl or acetyl), an amidino group (-C(=NH)-NH₂), or an N-mono- or N,N'-di(lower alkyl)-amidino group include 4-aminobutyl (related to lysine or its derivatives) and 3-amidino[or carbamidoyl (-C(=NH)-NH₂)]-aminopropyl (related to arginine or its derivatives).

Typical examples of substituted lower alkyl groups in which the substituent is a phenyl group that may optionally be substituted by a hydroxyl group or in which the substituent is a five-membered heterocyclic group that has one or two nitrogen atoms in the ring and may optionally have a benzene ring fused thereto include phenylmethyl (related to phenylalanine or its derivatives), 4-hydroxyphenylmethyl (related to tyrosine or its derivatives), (1H-imidazol-4-yl)methyl (related to histidine or its derivatives) and (1H-indol-3-yl)methyl (related to tryptophan or its derivatives).

Moreover, typical examples of substituted lower alkyl groups in which the substituent is a carbamoyl group (-CONH₂) include carbamoylmethyl (related to asparagine or its derivatives) and carbamoylethyl (related to glutamine or its derivatives).

The lower alkyl groups represented by R² and R³ may be the same lower alkyl groups as defined for R¹. The lower acyl groups represented by R² and R³ may be acyl groups corresponding to lower alkyl groups, such as formyl, acetyl and propionyl.

Moreover, the acyl groups represented by R² and R³ may also be intermediate or higher acyl groups. These acyl groups include residues derived from fatty acids having 7 to 26 carbon atoms. Preferably, such fatty acids are ones derived from natural lipids. Specific examples thereof include saturated fatty acids derived from rice bran oil, wax, lanolin, coconut oil, palm oil, milk fat and the like, such as cerotic acid, lignoceric acid, behenic acid, arachidic acid, stearic acid, palmitic acid, myristic acid, lauric acid, capric acid, caprylic acid, caproic acid and butyric acid; and unsaturated fatty acids derived from terrestrial animal fats (in particular, tallow), aquatic animal oils (in particular, fish liver oil), other vegetable oils (e.g., coriander oil and evening primrose seed oil) and the like, such as palmitoleic acid, petroselinic acid, oleic acid, elaidic acid, linolic acid, γ-linolenic acid and arachidonic acid. Moreover, these fatty acids may have a hydroxyl group, as is the case with ricinolic acid and α-hydroxylinolenic acid. Preferably, both R² and R³ represent hydrogen atoms.

In addition to the above-described definition, one of R² and R³ may be combined with R¹ to yield a residue which, together with the nitrogen and carbon atoms joined thereto, forms a five-membered ring. Specific examples of the residue include propane-1,3-diyl (-CH₂CH₂CH₂-) (related to proline or its derivatives), 2-hydroxypropane-1,3-diyl (-CH₂CH(OH)CH₂-) (related to 4-hydroxyproline or its derivatives) and 1-hydroxypropane-1,3-diyl (-CH(OH)CH₂CH₂-) (related to 3-hydroxyproline or its derivatives).

The above-described compounds may be salts formed through the medium of the carboxyl group especially when one of R² and R³ is a lower acyl group, or salts formed through the medium of the R²(R³)N- group when both R² and R³ are lower alkyl groups and the carboxyl group is esterified (i.e., in the form of a carboxylic acid ester). Typical examples of the salts formed through the medium of the carboxyl group include ammonium salts and alkali metal salts such as sodium, lithium and potassium salts. Typical examples of the salts formed through the medium of the R²(R³)N- group include salts formed with hydrochloric acid, hydrobromic acid, phosphoric acid, sulfuric acid, organic sulfonic acids (e.g., methanesulfonic acid and ethanesulfonic acid) and organic carboxylic acids (e.g., acetic acid, citric acid, malic acid, succinic acid and tartaric acid).

The carboxylic acid esters may be esters formed from a lower to higher alkanol (e.g., methanol, ethanol, octanol, decanol, stearyl alcohol or octacosanol) and the carboxyl group. Moreover, when both R² and R³ are lower alkyl groups (preferably methyl), compounds having a quaternary ammonium group formed by the joining of an additional lower alkyl group (preferably methyl) to the nitrogen atom to which the lower alkyl groups are attached (related to N,N,N-trimethylglycine or its derivatives) also fall within the scope of the active ingredient of the present invention.

Among the above-described compounds, those belonging to the category of naturally occurring α-amino acids may especially conveniently be used. However, with respect to α-amino acids other than glycine, L-amino acid derivatives, their enantiomers (D-isomers) and their racemates may also be conveniently used.

The above-defined compounds of formula (I) may be made into various preparations by compounding them with physiologically acceptable diluents, carriers, adjuvants and other active substances which are used in the field of medicines or cosmetics, provided that these preparations suit the purpose of the present invention. Typical examples of such diluents, carriers and adjuvants include, but are not limited to, medicinal preparations, antibacterial agents, pH adjustors, antioxidants, etc., such as fats and fatty oils including wax, hydrocarbon oils, higher fatty acids, higher alcohols, silcones, surfactants, alcohols, water, viscosity adjustors, chelating agents, ultraviolet light absorbers, humectants and skin activators.

Examples of oils and fatty oils include linseed oil, tsubaki oil, macadamia nut oil, corn oil, mink oil, olive oil, avocado oil, sasanqua oil, castor oil, safflower oil, apricot oil, cinnamon oil, jojoba oil, grape oil, sunflower oil, almond oil, rapeseed oil, sesame oil, wheat germ oil, rice germ oil, rice bran oil, cottonseed oil, soybean oil, peanut oil, teaseed oil, evening primrose oil, eggyolk oil, neat's foot oil, liver oil, triglycerine, glycerine trioctanate, glycerine triisopalmitate, and other liquid oils and fats; coconut oil, palm oil, palm kernel oil, and other liquid or solid oils and fats; cacao fat, beef tallow, sheep fat, hog fat, horse fat, hydrogenated oil, hydrogenated castor oil, Japanese wax, Shea butter, and other solid oils and fats; beeswax, candelilla wax, cotton wax, carnauba wax, bayberry wax, tree wax, spermaceti, montan wax, bran wax, lanolin, reduced lanolin, hard lanolin, kapok wax, sugarcane wax, jojoba wax, shellac wax, and other waxes.

Further, it is also possible to formulate, as a basis, into the preparation of the present invention cetyl octanate and other octanic acid esters, glyceryl tri-2-ethylhexanoate, pentaerythritol tetra-2-ethylhexanoate, and other isooctanic acid esters; hexyl laurate and other lauric acid esters, isopropyl myristate, octyldodecyl myristate, and other myristic acid esters, octyl palmitate and other palmitic acid esters, isocetyl stearate and other stearic acid esters, isopropyl isostearate and other isostearic acid esters, octyl isopalmitate and other isopalmitic acid esters, isodecyl oleate and other oleic acid esters, diisopropyl adipate and other adipic acid diesters, diethyl sebacate and other sebacic acid diesters, diisostearyl malate, and other ester oils; liquid paraffin, ozocerite, squalane, squalene, pristane, paraffin, isoparaffin, ceresin, vaseline, microcrystalline wax, and other hydrocarbon oils, etc.

As a higher fatty acid, for example, lauric acid, myristic acid, palmitic acid, stearic acid, behenic acid, oleic acid, 12-hydroxystearic acid, undecylic acid, toluic acid, isostearic acid, linolic acid, linoleic acid, eicosapentaenoic acid (EPA), docosahexaenoic acid (DHA), etc. may be mentioned

As a higher alcohol, for example, lauryl alcohol, cetyl alcohol, stearyl alcohol, behenyl alcohol, myristyl alcohol, oleyl alcohol, cetostearyl alcohol, and other straight chain alcohols, monostearyl glycerin ether (batyl alcohol), 2-decyltetradecinol, lanolin alcohol, cholesterol, phytosterol, hexyldodecanol, isostearyl alcohol, octyldodecanol, and other branched chain alcohols etc. may be mentioned.

It is also possible to formulate, as further basis, into the preparation of the present invention dimethyl polysiloxane, methylphenyl polysiloxane, methylhydrogen polysiloxane, and other chain like siloxanes, octamethylcyclotetrasiloxane, decamethylcyclopentasiloxane, dodecamethylcyclohexasiloxane, and other cyclic siloxanes, silicone resins having a three-dimensional network structures, silicone rubber, etc.

Examples of surfactants include soap base, sodium laurate, sodium palmitate, and other fatty acid soaps, sodium laurosulfate, potassium laurosulfate, and other higher alkyl sulfate ester salts, POE laurosulfate triethanol amine, sodium POE laurosulfate, and other alkyl ether sulfate ester salts, sodium lauroylsarcosine and other N-acylsarcosine acids, sodium N-myristyl-N-methyltaurine, sodium N-cocoyl-N-methyl taurate, sodium laurylmethyl taurate, and other higher fatty acid amide sulfonates, sodium POE oleyl ether phosphate, POE stearyl ether phosphate, and other phosphate ester salts, sodium di-2-ethylhexylsulfosuccinate, sodium monolauroylmonoethanol amide polyoxyethylene sulfosuccinate, sodium laurylpolypropylene glycol sulfosuccinate, and other sulfosuccinates, linear sodium dodecylbenzensulfonate, linear dodecylbenzensulfonate triethanol amine, linear dodecyl benzensulfate, and other alkylbenzensulfonates, monosodium N-lauroylglutamate, disodium N-stearoylglutamate, monosodium N-myristoyl-L-glutamate, and other N-acylglutamates, sodium hydrogenated coconut oil fatty acid glycerin sulfate and other higher fatty acid ester sulfate ester salts, Turkey red oil and other sulfated oils, POE alkyl ether carboxylic acid, POE alkylaryl ether carboxylate, α-olefinsulfates, higher fatty acid ester sulfonates, secondary alcohol sulfate ester salts, higher fatty acid alkylolamide sulfate ester salts, sodium lauroyl monoethanolamide succinate, N-palmitoyl asparaginate ditriethanol amine, sodium caseine, and other anionic surfactants;
Stearyl trimethyl ammonium chloride, lauryl trimethyl ammonium chloride, and other alkyl trimethyl ammonium salts, distearyldimethyl ammonium chloride, dialkyldimethyl ammonium chloride salts, poly(N,N'-dimethyl-3,5-methylenepiperidinium)chloride, cetylpyridinium chloride and other alkyl pyridinium salts, alkyl quaternary ammonium salts, alkyl dimethylbenzyl ammonium salts, alkyl isoquinolinium salts, dialkyl morphonium salts, POE alkyl amines, alkyl amine salts, polyamine fatty acid derivatives, amyl alcohol fatty acid derivatives, benzalkonium chloride, benzethonium chloride, and other cationic surfactants; sodium 2-undecyl-N,N,N-(hydroxyethylcarboxymethyl)-2-imidazoline, 2-cocoyl-2-imidazoliniumhydroxide-1-carboxyethyloxy-2-sodium salt, and other imidazoline family bipolar surfactants, 2-heptadecyl-N-carboxynethyl-N-hydroxyethylimidazolinium betaine, lauryldimethyl-aminoacetate betaine, alkyl betaine, amide betaine, sulfo betaine, and other betaine family surfactants, and other bipolar surfactants;
Sorbitan monooleate, sorbitan monoisostearate, sorbitan monolaurate, sorbitan monopalmitate, sorbitan monostearate, sorbitan sesquioleate, sorbitan trioleate, diglyceryl sorbitan pentaoctanoate, diglyceryl sorbitan tetraoctanoate, and other sorbitan fatty acid esters, glycerin mono cotton seed oil fatty acid, glycerin monoerucate, glycerin sesquioleate, glycerin monostearate, glycerin α,α'-oleate pyroglutamate, monostearate glycerin malic acid and other glycerin polyglycerin fatty acids, propylene glycol monostearate and other propylene glycol fatty acid esters, hydrogenated eastor oil derivatives, glycerin alkyl ethers, polyoxyethylene methylpolysiloxane copolymers, and other lyophilic nonionic surfactants;
POE sorbitan monooleate, POE sorbitan monostearate, POE-sorbitan monooleate, POE-sorbitan tetraoleate, and other POE sorbitan fatty acid esters, POE-sorbit monolaurate, POE-sorbit monooleate, POE-sorbit pentaoleate, POE-sorbit monostearate, and other POE sorbit fatty acid esters, POE-glycerin monostearate, POE-glycerin monoisostearate, POE-glycerin triisostearate, and other POE glycerin fatty acid esters, POE monooleate, POE distearate, POE monodioleate, distearate ethylene glycol, and other POE fatty acid esters, POE lauryl ethers, POE oleyl ethers, POE stearyl ethers, POE behenyl ethers, POE2-Octyldodecyl ethers, POE cholestanol ethers, and other POE alkyl ethers, POE octyl phenyl ethers, POE nonyl phenyl ethers, POE dinonyl phenyl ethers, and other POE alkyl phenyl ethers, Pluronic and other pluaronics, POE·POP cetyl ethers, POE·POP-2-decyltetradecyl ethers, POE·POP monobutyl ethers, POE·POP hydrated lanolin, POE·POP glycerin ethers, and other POE-POP alkyl ethers, Tetronic and other tetra-POE·tetra-POP ethylene diamine condensation products, POE castor oil, POE hydrogenated castor oil, POE hydrogenated castor oil monoisostearate, POE hydrogenated castor oil triisostearate, POE hydrogenated castor oil monopyroglutamate monoisostearate diester, POE hydrogenated castor oil maleic acid and other POE castor oil hydrogenated castor oil derivatives, POE sorbit beeswax and other POE beeswax lanolin derivatives, coconut oil fatty acid diethanolamide, laurate monoethanolamide, fatty acid isopropanolamide, and other alkanolamides, POE propylene glycol fatty acid esters, POE alkylamines, POE fatty acid amides, sucrose fatty acid esters, POE nonylphenyl formaldehyde condensation products, alkylethoxydimethylamineoxide, trioleylphosphoric acid, and other hydrophilic nonionic surfactants and other surfactants.

Examples of alcohols include methanol, ethanol, propanol, isopropanol, and other lower alcohols, cholesterols, cytosterols, phytosterols, lanosterols, and other sterols, etc.

Examples of viscosity adjustor include arabia gum, tragacanth, galactan, carob gum, guar gum, karaya gum, carragheenin, pectin, agar, quince, seed, algae colloids (algae extract), starch (rice, corn, potato, wheat), and other plant type polymers, dextran, succinoglucan, pulleran, and other microbial type polymers, carboxymethyl starch, methylhydroxypropyl starch, and other starch type polymers, collagen, casein, albumin, gelatin, and other animal type polymers, methyl cellulose, nitrocellulose, ethyl cellulose, methylhydroxypropyl cellulose, hydroxyethyl cellulose, sodium cellulose sulfate, hydroxypropyl cellulose, sodium carboxymethyl cellulose, crystalline cellulose, cellulose powder, and other cellulose type polymers, sodium alginate, alginic acid propylene glycol esters and other alginic acid type polymers, polyvinylmethyl ethers, carboxyvinyl polymers (CARBOPOL etc.), and other vinyl type polymers, polyoxyethylene type polymers, polyoxy-ethylene-polyoxypropylene copolymer type polymers, sodium polyacrylate, polyethylacrylate, polyacrylamide, and other acryl type polymers, polyethyleneimine, cation polymers, bentonite, aluminum magnesium silicate, laponite, hectorite, anhydrous silicic acid, and other inorganic type water-soluble polymers, etc.

Examples of chelating agents include citramalic acid, agaricic acid, glyceric acid, lactobionic acid, dihydroxyfumaric acid, quinic acid, galactaric acid, glucaric acid, shikimic acid, ascorbic acid, hinokitiol, ginkgolic acid, homogentisic acid, protocatechuic acid, gallic acid, tannic acid, caffeic acid, meconic acid, gentisic acid, α-kainic acid, ethylenediamine tetraacetic acid, 1,2-bis(2-aminophenoxy)ethanN,N,N',N'-tetraacetic acid, ethyleneglycol diamine tetraacetic acid, diethylene triamine pentaacetic acid, orotic acid, N-(2-hydroxyethyl)ethylene diamine triacetic acid, α-glycerophosphoric acid, β-glycerophosphoric acid, phytic acid, trimellitic acid, phosphoric acid, polyphosphoric acid and metaphosphoric acid, and compounds which are functionally similar to the above, and, if suitable, alkali metal salts and carboxylic acid esters of the above-mentioned compounds.

Examples of UV absorbants include p-amino-benzoic acid and other benzoic acid-type UV absorbants, methyl anthranilate and other anthranilic acid-type UV absorbants, octyl salicylate, phenyl salicylate, homomethyl salicylate, and other salicylic acid-type UV absorbants, isopropyl p-methoxycinnamate, octyl p-methoxycinnamate, 2-ethylhexyl p-methoxycinnamate, glyceryl di-p-methoxycinnamate mono-2-ethylhexanoate, [4-bis(trimethylsiloxy) methylsilyl-3-methylbutyl]-3,4,5-trimethoxycinnamic acid esters and other cinnamic acid-type UV absorbants, 2,4-dihydroxybenzophenone, 2-hydroxy-4-methoxybenzophenone, 2-hydroxy-4-methoxybenzophenone-5-sulfonic acid, sodium 2-hydroxy-4-methoxybenzophenone-5-sulfonic acid, and other benzophenone-tyke UV absorbants, urocanic acid, ethyl urocanate, 2-phenyl-5-methylbenzoxazole, 2-(2'-hydroxy-5'-methylphenyl)benzotriazole, 4-tert-butyl-4'-methoxybenzoylmethane, etc.

Examples of humectants include polyethylene glycol, propylene glycol, dipropylene glycol, 1,3-butylene glycol, hexylene glycol, glycerine, diglycerine, xylitol, maltitol, maltose, D-mannitol, saccharified starch, glucose, fructose, lactose, sodium chondroitin sulfate, sodium hyaluronate, sodium adenosine phosphate, sodium lactate, gallates, pyrrolidone carbonates, glucosamine, cyclodextrin, etc.

Examples of medicinal ingredients include vitamin A oil, retinol, retinol palmitate, inositol, pyridoxine chloratel, benzyl nicotinate, nicotinamide, dl-α-tocopheryl nicotinate, magnesium ascorbyl phosphate, vitamin D₂ (ergocalciferol), dl-α-tocopherol, potassium dl-α-tocopherol-2-L-ascorbic diester, dl-α-tocopheryl acetate, pantothenic acid, biotin, and other vitamins, estradiol, ethynylestradiol and other hormones, allantoin, azulene, glycyrrhetinic acid, and other antiinflammatories, arbutin and other whiteners, zinc oxide, tannic acid, and other astringents, L-menthol, camphor, and other fresheners or sulfur, lysozyme chloride, pyridoxine chlorate, γ-oryzanol, etc. Further, it is possible to formulate various types of extracts having various medicinal effects such as Houttuynia cordate extract, Phellon dendron amurense Rupr extract, melilot extract, white dead nettle extract, licorice root extract, herbaceous peony extract, soapwort extract, dishcloth gourd extract, cinchona extract, creeping saxifrage extract, Sophora angustifolia extract, candock extract, common fennel extract, primrose extract, rose extract, Rehmannia glutinosa extract, lemon extract, Lithospermum erythrorhizon extract, aloe extract, iris rhizome extract, eucalyptus extract, field horsetail extract, sage extract, thyme extract, tea extract, seaweed extract, cucumber extract, clove extract, raspberry extract, melissa extract, carrot extract, horse chestnut extract, peach extract, peach leaf extract, mulberry extract, cornflower extract, hamamelis extract, placenta extract, thymus extract, silk extract, etc.

Examples of antibacterial agents include benzoic acid, salicylic acid, carbolic acid, sorbic acid, p-oxybenzoic acid esters, p-chlorometacresol, hexachlorophene, benzalkonium chloride, chlorohexidine chloride, trichlorocarbanilide, photosensitive element, phenoxyethanol.

Furthermore, the preparation of the present invention may contain 2-amino-2-methyl-1-propanol, 2-amino-2-methyl-1,3-propanediol, potassium hydroxide, sodium hydroxide, triethanolamine, sodium carbonate, and other neutralizing agents; lactic acid, citric acid, glycolic acid, succinic acid, tartaric acid, dl-malic acid, potassium carbonate, sodium hydrogencarbonate, ammonium hydrogencarbonate, and other pH adjustors; ascorbic acid, α-tocopherol, dibutylhydroxytoluene, butylhydroxyanisole, and other antioxidants; and preservatives such as methylparaben, ethylparaben and butylparaben.

Further, if necessary, it is also possible to formulate into the preparation of the present invention suitable powder components, perfume, color, etc. to such an extent as not to impair the desired effect of the present invention.

Here, the above ingredients are all examples only. The ingredients which may be formulated into the preparation of the present invention are not limited to these ingredients.

These ingredients may be formulated into the preparation of the present invention in any formulation according to the desired form, and in combination appropriately.

The preparation of the present invention which is prepared by compounding the above-mentioned effective ingredients with diluents, carriers and the like may be broadly used in the form of a pharmaceutical, a quasi-drug (ointment etc.) and cosmetic [facial cleanser, emulsion, cream, gel, essence (beauty liquid), pack, mask or other basic cosmetic; foundation, lipstick or other makeup cosmetic; aromatic cosmetic and body cosmetic].

The preparation of the present invention may take broad range of properties and types such as aqueous solution system, solubilized system, emulsified system, powder system, oily liquid system, gel system, ointment system, air sol system, water-oil two-layer system, water-oil-powder three-layer system, etc.

In external preparations for application to the skin, or preparations for enhancing the stratum corneum desquamation or desmosomal degradation of the skin, in accordance with the present invention, the compound of formula (I) may be used in an effective amount of 0.01 to 20% by weight, depending on the properties and physiological activity of the compound. These preparations are mainly administered by direct application to the skin, and the dosage of the compound of formula (I) may be suitably controlled according to the age of the subject being treated and the severity of symptoms presented by the skin.

Thus, the preparations of the present invention makes it possible to prevent or treat a morbid thickening of the skin. Moreover, in normal subjects, they can harmonize the formation of the stratum corneum with its shedding by physiological desquamation and thereby maintain a fresh and young skin condition.

The present invention is more fully explained with reference to the following specific examples.

### Method of evaluation (the percentage of residual desmoglein in stratum corneum sheets)

This method of evaluation serves to evaluate the degree of decomposition of desmoglein in a stratum corneum sheet by trypsin-like and chymotrypsin-like enzymes, i.e., the degree of stratum corneum desquamation. Lower percentages of residual desmoglein indicate that the activities of the aforesaid enzyme are more strongly enhanced.

Stratum corneum sheets were peeled from healthy subjects and soaked in an antiseptic and fungicidal solution (containing 60 µl/ml of kanamycin and 0.5% NaN₃) for 30 seconds. Both sides of a stratum corneum sheet weighing 2 mg were coated with 5 µl of a 5% aqueous solution containing each of the test compounds shown in Table I below. A stratum corneum sheet coated with an aqueous solution containing no test compound was regarded as a control. These stratum corneum sheets were subjected to the following procedure.

The stratum corneum sheets prepared in the above-described manner were placed under such conditions that the stratum corneum would have a moisture content of not greater than 30% at which the degradation of desmosomes was insufficient, and allowed to stand at 37°C for one week. Thereafter, using 0.5 ml of a 0.1M Tris buffer (pH 9) containing 9M urea, 2% SDS and 1% mercaptoethanol, each of the stratum corneum sheets was extracted at 37°C for 15 hours. Then, 0.7 ml of a sample buffer for SDS-PAGE (a two-fold concentrated Laemmli solution) was added to the resulting extract and heated for 15 minutes. 10 µl of this solution was taken and subjected to electrophoresis in a gel having a concentration of 7.5%. After electrophoresis, the proteins were transferred to a PVDF membrane and immunologically stained with an anti-desmoglein antibody and alkaline phosphatase-labelled secondary (anti-mouse Ig) antibody. Then, the labelled protein was visualized with NBT/BCIP substrate. Thus, the amount of the desired protein was determined. The results are expressed by the average value of three measurements and summarized in Table I below.

**Table I**

| Test compound | Percentage of residual desmoglein |
|---|---|
| Control | 100.00 |
| Glycine | 84.13 |
| DL-Alanine | 54.55 |
| DL-Serine | 69.44 |
| L-Serine | 58.41 |
| DL-Threonine | 97.50 |
| L-Threonine | 90.91 |
| 4-Hydroxy-L-proline | 83.04 |
| N,N,N-Trimethylglycine | 86.08 |
| N-Dodecanoylglycine potassium salt | 78.58 |

Then, the above evaluation process was repeated except that the above-mentioned 5 % aqueous solution of each of the test compounds was replaced with an aqueous solution which had been adjusted to contain 0.1 % of L-alanine and 2.9 % of N,N,N-trimethylglycine. As a result, percentage of residual desmoglein was found to be 65.4 %.

### Preparation Example 1: Toilet water

### (Formulation)

| Ingredient | wt.% |
|---|---|
| 1,3-Butylene glycol | 6.0 |
| Glycerin | 4.0 |
| Oleyl alcohol | 0.1 |
| POE (20) sorbitan monolaurate | 0.5 |
| POE (15) lauryl alcohol ether | 0.5 |
| Ethanol | 10.0 |
| DL-Alanine | 10.0 |
| Purified water | 68.9 |

### (Preparation method)

1,3-Butylene glycol and glycerin were dissolved in purified water at room temperature to form an aqueous phase. Other ingredients were dissolved in ethanol, and the resulting solution was mixed with the above aqueous phase to solubilize these ingredients. Then, the resulting mixture was filtered and packed to obtain toilet water.

### Preparation Example 2: Toilet water

### (Formulation)

| Ingredient | wt.% |
|---|---|
| 1,3-Butylene glycol | 6.0 |
| Glycerin | 4.0 |
| Oleyl alcohol | 0.1 |
| POE (20) sorbitan monolaurate | 0.5 |
| POE (15) lauryl alcohol ether | 0.5 |
| Ethanol | 10.0 |
| L-Serine | 1.0 |
| Purified water | 77.9 |

### (Preparation method)

1,3-Butylene glycol and glycerin were dissolved in purified water at room temperature to form an aqueous phase. Other ingredients were dissolved in ethanol, and the resulting solution was mixed with the above aqueous phase to solubilize these ingredients. Then, the resulting mixture was filtered and packed to obtain toilet water.

### Preparation Example 3: Toilet water

### (Formulation)

| Ingredient | wt.% |
|---|---|
| 1,3-Butylene glycol | 6.0 |
| Glycerin | 4.0 |
| Oleyl alcohol | 0.1 |
| POE (20) sorbitan monolaurate | 0.5 |
| POE (15) lauryl alcohol ether | 0.5 |
| Ethanol | 10.0 |
| DL-Serine | 0.1 |
| N,N,N-Trimethylglycine | 0.5 |
| Purified water | 78.3 |

### (Preparation method)

1,3-Butylene glycol and glycerin were dissolved in purified water at room temperature to form an aqueous phase. Other ingredients were dissolved in ethanol, and the resulting solution was mixed with the above aqueous phase to solubilize these ingredients. Then, the resulting mixture was filtered and packed to obtain toilet water.

### Preparation Example 4: Toilet water

### (Formulation)

| Ingredient | wt.% |
|---|---|
| 1,3-Butylene glycol | 6.0 |
| Glycerin | 4.0 |
| Oleyl alcohol | 0.1 |
| POE (20) sorbitan monolaurate | 0.5 |
| POE (15) lauryl alcohol ether | 0.5 |
| Ethanol | 10.0 |
| DL-Alanine | 0.1 |
| L-Serine | 1.0 |
| Purified water | 77.8 |

### (Preparation method)

1,3-Butylene glycol and glycerin were dissolved in purified water at room temperature to form an aqueous phase. Other ingredients were dissolved in ethanol, and the resulting solution was mixed with the above aqueous phase to solubilize these ingredients. Then, the resulting mixture was filtered and packed to obtain toilet water.

### Preparation Example 5: Cream

### (Formulation)

| Ingredient | wt.% |
|---|---|
| (1) Stearyl alcohol | 6.0 |
| (2) Stearic acid | 2.0 |
| (3) Hydrogenated lanolin | 4.0 |
| (4) Squalane | 9.0 |
| (5) Octyldodecanol | 10.0 |
| (6) 1,3-Butylene glycol | 6.0 |
| (7) Polyethylene glycol 1500 | 4.0 |
| (8) POE (25) cetyl alcohol ether | 3.0 |
| (9) Glyceryl monostearate | 2.0 |
| (10) L-Serine | 1.0 |
| (11) Tocopherol | 0.1 |
| (12) Purified water | 59.9 |

### (Preparation method)

Ingredients (6) and (7) were added to purified water (12), and the resulting solution was heated and adjusted to 70°C. Ingredients (1) to (5) were melted by heating, ingredients (8) to (11) were added thereto, and the resulting mixture was adjusted to 70°C. This mixture was added to the above aqueous phase and homogenized with a homomixer so as to give uniform particles. The resulting emulsion was degassed, filtered and cooled to obtain a cream.

### Preparation Example 6: Toilet water

### (Formulation)

| Ingredient | wt.% |
|---|---|
| (1) Sorbitol | 4.0 |
| (2) Dipropylene glycol | 6.0 |
| (3) Polyethylene glycol | 5.0 |
| (4) POE (20) oleyl alcohol ether | 0.5 |
| (5) Methylcellulose | 0.2 |
| (6) Quince seed | 0.1 |
| (7) Ethanol | 10.0 |
| (8) Perfume | q.s. |
| (9) Paraben | 0.1 |
| (10) Sodium phosphate | 1.0 |
| (11) DL-Alanine | 5.0 |
| (12) Purified water | 68.1 |

### (Preparation method)

Ingredients (5) and (6) were added to part of purified water (12) and stirred to form a viscous solution. Ingredients (1), (2), (3), (10) and (11) were added to the remainder of purified water (12) and dissolved therein at room temperature, followed by the addition of the above viscous solution. Thus, there was obtained a homogeneous aqueous solution. Ingredients (9), (4) and (8) were added to ethanol (7) and dissolved therein to form an alcoholic solution. This alcoholic solution was added to and mixed with the above aqueous solution. Thus these ingredients were solubilized to obtain toilet water.

### Preparation Example 7: Lotion

### (Formulation)

| Ingredient | wt.% |
|---|---|
| (1) Stearic acid | 2.0 |
| (2) Cetyl alcohol | 1.5 |
| (3) Petrolatum | 4.0 |
| (4) Squalane | 5.0 |
| (5) Glycerol tri-2-ethylhexanoate | 2.0 |
| (6) Sorbitan monooleate | 6.0 |
| (7) Dipropylene glycol | 4.0 |
| (8) Polyethylene glycol 1500 | 3.0 |
| (9) Triethanolamine | 2.0 |
| (10) Paraben | 1.0 |
| (11) Perfume | q.s. |
| (12) DL-Serine | 5.0 |
| (13) Purified water | 69.4 |

### (Preparation method)

Ingredients (7), (8) and (9) were added to purified water, and the resulting solution was heated and adjusted to 70°C. Ingredients (1) to (5) were melted, ingredients (6), (10), (11) and (12) were added thereto, and the resulting mixture was adjusted to 70°C. This oily phase was added to the previously prepared aqueous phase and subjected to preliminary emulsification. Then, this mixture was homogenized with a homomixer so as to give uniform particles. The resulting emulsion was degassed, filtered and cooled to obtain a lotion.

### Preparation Example 8: Cleansing foam

### (Formulation)

| Ingredient | wt.% |
|---|---|
| (1) Stearic acid | 12.0 |
| (2) Myristic acid | 14.0 |
| (3) Lauric acid | 5.0 |
| (4) Jojoba oil | 3.0 |
| (5) Potassium hydroxide | 5.0 |
| (6) Sorbitol (70% solution) | 15.0 |
| (7) Glycerin | 10.0 |
| (8) 1,3-Butylene glycol | 10.0 |
| (9) POE (20) glycerol monostearate | 2.0 |
| (10) Acyl methyltaurine | 4.0 |
| (11) L-Seine | 0.5 |
| (12) Perfume | q.s. |
| (13) Purified water | 20.0 |

### (Preparation method)

Ingredients (1) to (4), (6) to (8), and (11) were melted by heating, and held at 70°C. Ingredient (5) was dissolved in purified water, and the resulting solution was added to the oily phase with stirring. After the neutralization reaction was effected for a sufficient period of time, surfactants (9) and (10) were added thereto, followed by the addition of ingredient (12). The resulting mixture was degassed, filtered and cooled to obtain a cleansing foam.

### Preparation Example 9: Lotion

### (Formulation)

| Ingredient | wt.% |
|---|---|
| (1) Stearic acid | 2.0 |
| (2) Cetyl alcohol | 1.5 |
| (3) Petrolatum | 4.0 |
| (4) Squalane | 5.0 |
| (5) Glycerol tri-2-ethylhexanoate | 2.0 |
| (6) Sorbitan monooleate | 6.0 |
| (7) Dipropylene glycol | 4.0 |
| (8) Polyethylene glycol 1500 | 3.0 |
| (9) Triethanolamine | 2.0 |
| (10) Paraben | 1.0 |
| (11) Perfume | q.s. |
| (12) DL-Alanine | 0.2 |
| (13) N,N,N-Trimethylglycine | 3.0 |
| (14) Purified water | 71.2 |

### (Preparation method)

Ingredients (7), (8) and (9) were added to purified water, and the resulting solution was heated and adjusted to 70°C. Ingredients (1) to (5) were melted, ingredients (6), (10), (11), (12) and (13) were added thereto, and the resulting mixture was adjusted to 70°C. This oily phase was added to the previously prepared aqueous phase and subjected to preliminary emulsification. Then, this mixture was homogenized with a homomixer so as to give uniform particles. The resulting emulsion was degassed, filtered and cooled to obtain a lotion.

### Preparation Example 10: Toilet water

### (Formulation)

| Ingredient | wt.% |
|---|---|
| 1,3-Butylene glycol | 6.0 |
| Glycerin | 4.0 |
| Oleyl alcohol | 0.1 |
| POE (20) sorbitan monolaurate | 0.5 |
| POE (15) lauryl alcohol ether | 0.5 |
| Ethanol | 10.0 |
| DL-Alanine | 0.1 |
| N,N,N-Trimethylglycine | 3.0 |
| N-(2-hydroxyethyl)ethylene diaminetriacetic acid | 0.1 |
| Purified water | 75.7 |

### (Preparation method)

1,3-Butylene glycol and glycerin were dissolved in purified water at room temperature to form an aqueous phase. Other ingredients were dissolved in ethanol, and the resulting solution was mixed with the above aqueous phase to solubilize these ingredients. Then, the resulting mixture was filtered and packed to obtain toilet water.

### Preparation Example 11: Toilet water

### (Formulation)

| Ingredient | wt.% |
|---|---|
| 1,3-Butylene glycol | 6.0 |
| Glycerin | 4.0 |
| Oleyl alcohol | 0.1 |
| POE (20) sorbitan monolaurate | 0.5 |
| POE (15) lauryl alcohol ether | 0.5 |
| Ethanol | 10.0 |
| L-Serine | 0.3 |
| N,N,N-Trimethylglycine | 1.0 |
| N-(2-hydroxyethyl)ethylene diaminetriacetic acid | 0.2 |
| Purified water | 77.4 |

### (Preparation method)

1,3-Butylene glycol and glycerin were dissolved in purified water at room temperature to form an aqueous phase. Other ingredients were dissolved in ethanol, and the resulting solution was mixed with the above aqueous phase to solubilize these ingredients. Then, the resulting mixture was filtered and packed to obtain toilet water.

### Preparation Example 12: Toilet water

### (Formulation)

| Ingredient | wt.% |
|---|---|
| 1,3-Butylene glycol | 6.0 |
| Glycerin | 4.0 |
| Oleyl alcohol | 0.1 |
| POE (20) sorbitan monolaurate | 0.5 |
| POE (15) lauryl alcohol ether | 0.5 |
| Ethanol | 10.0 |
| DL-Alanine | 0.5 |
| N-(2-hydroxyethyl)ethylene diaminetriacetic acid | 0.1 |
| Purified water | 78.3 |

### (Preparation method)

1,3-Butylene glycol and glycerin were dissolved in purified water at room temperature to form an aqueous phase. Other ingredients were dissolved in ethanol, and the resulting solution was mixed with the above aqueous phase to solubilize these ingredients. Then, the resulting mixture was filtered and packed to obtain toilet water.

### Preparation Example 13: Toilet water

### (Formulation)

| Ingredient | wt.% |
|---|---|
| 1,3-Butylene glycol | 6.0 |
| Glycerin | 4.0 |
| Oleyl alcohol | 0.1 |
| POE (20) sorbitan monolaurate | 0.5 |
| POE (15) lauryl alcohol ether | 0.5 |
| Ethanol | 10.0 |
| L-Serine | 0.5 |
| N-(2-hydroxyethyl)ethylene diaminetriacetic acid | 0.2 |
| Purified water | 78.2 |

### (Preparation method)

1,3-Butylene glycol and glycerin were dissolved in purified water at room temperature to form an aqueous phase. Other ingredients were dissolved in ethanol, and the resulting solution was mixed with the above aqueous phase to solubilize these ingredients. Then, the resulting mixture was filtered and packed to obtain toilet water.

### Preparation Example 14: Toilet water

### (Formulation)

| Ingredient | wt.% |
|---|---|
| 1,3-Butylene glycol | 6.0 |
| Glycerin | 4.0 |
| Oleyl alcohol | 0.1 |
| POE (20) sorbitan monolaurate | 0.5 |
| POE (15) lauryl alcohol ether | 0.5 |
| Ethanol | 10.0 |
| N,N,N-Trimethylglycine | 5.0 |
| N-(2-hydroxyethyl)ethylene diaminetriacetic acid | 1.0 |
| Purified water | 72.9 |

### (Preparation method)

1,3-Butylene glycol and glycerin were dissolved in purified water at room temperature to form an aqueous phase. Other ingredients were dissolved in ethanol, and the resulting solution was mixed with the above aqueous phase to solubilize these ingredients. Then, the resulting mixture was filtered and packed to obtain toilet water.

### Preparation Example 15: Toilet water

### (Formulation)

| Ingredient | wt.% |
|---|---|
| 1,3-Butylene glycol | 6.0 |
| Glycerin | 4.0 |
| Oleyl alcohol | 0.1 |
| POE (20) sorbitan monolaurate | 0.5 |
| POE (15) lauryl alcohol ether | 0.5 |
| Ethanol | 10.0 |
| L-Serine | 0.1 |
| DL-Alanine | 0.5 |
| N-(2-hydroxyethyl)ethylene diaminetriacetic acid | 0.2 |
| Purified water | 78.1 |

### (Preparation method)

1,3-Butylene glycol and glycerin were dissolved in purified water at room temperature to form an aqueous phase. Other ingredients were dissolved in ethanol, and the resulting solution was mixed with the above aqueous phase to solubilize these ingredients. Then, the resulting mixture was filtered and packed to obtain toilet water.

### Preparation Example 16: Toilet water

### (Formulation)

| Ingredient | wt.% |
|---|---|
| 1,3-Butylene glycol | 6.0 |
| Glycerin | 4.0 |
| Oleyl alcohol | 0.1 |
| POE (20) sorbitan monolaurate | 0.5 |
| POE (15) lauryl alcohol ether | 0.5 |
| Ethanol | 10.0 |
| L-Serine | 0.1 |
| DL-Alanine | 0.1 |
| N,N,N-Trimethylglycine | 1.0 |
| N-(2-hydroxyethyl)ethylene diaminetriacetic acid | 0.5 |
| Purified water | 78.2 |

### (Preparation method)

1,3-Butylene glycol and glycerin were dissolved in purified water at room temperature to form an aqueous phase. Other ingredients were dissolved in ethanol, and the resulting solution was mixed with the above aqueous phase to solubilize these ingredients. Then, the resulting mixture was filtered and packed to obtain toilet water.

## Claims

1. The use of a cosmetic or dermatological topical preparation containing, as active ingredient, at least one α-amino acid derivative of the following formula (I) that may be in the form of a salt, a carboxylic acid ester, or a quaternary ammonium compound when both R² and R³ are lower alkyl groups, for the purpose of enhancing the stratum corneum desquamation or desmosomal degradation of the skin, and/or preventing and decreasing the dullness of the skin: 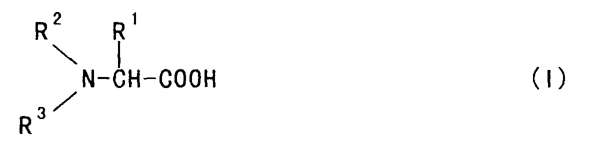
wherein R¹ is a hydrogen atom or an unsubstituted or substituted lower alkyl group, and the substituent in the substituted lower alkyl group is a hydroxyl group, a mercapto group which may optionally be substituted by a lower alkyl group, an amino group which may optionally be substituted by a lower alkyl group, a lower acyl group, an amid no group, or an N-mono- or N,N'-di(lower alkyl)amidino group, a phenyl group which may optionally be substituted by a hydroxyl group, a five-membered heterocyclic group which has one or two nitrogen atoms in the ring and which may optionally have a benzene ring fused thereto, or a carbamoyl group; and
R² and R³ are each independently a hydrogen atom, a lower alkyl group, or a lower, intermediate or higher acyl group; or
one of R² and R³ is combined with R¹ to form a propane-1,3-diyl, 2-hydroxypropane-1,3-diyl or 1-hydroxypropane-1,3-diyl group.

2. The use according to claim 1 wherein the α-amino acid derivative is selected from the group consisting of naturally occurring α-amino acids and racemates thereof.

3. The use according to claim 1 or 2 wherein R² and R³ in formula (I) are each independently a hydrogen atom, a lower alkyl group or a lower acyl group.

4. The use according to claim 1 or 2 wherein the α-amino acid derivative is an α-amino acid selected from the group consisting of alanine, valine, leucine, isoleucine, serine, threonine, cysteine, methionine, lysine, arginine, phenylalanine, tyrosine, histidine, tryptophan, asparagine, glutamine and proline, or a derivative of the α-amino acid.

5. The use according to any of claims 1 to 4 wherein the preparation is used to enhance the stratum corneum desquamation of the skin.

6. The use according to any of claims 1 to 4 wherein the preparation is used to enhance the desmosomal degradation of the skin.
